# EUROPEAN PATENT APPLICATION

(11) **EP 2 730 261 A1**
(43) Date of publication of application: **14.05.2014**
(21) Application number: 13192375.7
(22) Date of filing: 11.11.2013
(51) Int. Cl.: A61F 13/532, A61F 13/15, B32B 5/02, B32B 5/04, B32B 5/26, B32B 7/12, B32B 3/26

(54) **Apparatus and methods for forming laminates containing additive matter**

(30) Priority: 09.11.2012 US 201261724716 P
(71) Applicant: Curt G. Joa, Inc., Sheboygan Falls, Wisconsin 53085 (US)
(72) Inventor: Handziak,, Perry, Belgium, WI Wisconsin 53004 (US)
(74) Representative: Potter Clarkson LLP

(57) **Abstract**

Discrete or continuous products are disclosed including formation of laminates containing an additive matter. Such additive matters comprise gels, superabsorbent polymers, particulate additives and the like. Pulp-free absorbent cores for use in disposable products such as diapers and sanitary napkins can be formed by the methods and apparatus of the present invention. Additive matter is carried between two layers in void spaces in a scrim layer to form a laminate.

## Description

### Related Applications

This application claims the benefit of co-pending U.S. Provisional Patent Application Serial No. 61/724,716, filed 9 November 2012.

### Background of the Invention

This invention relates to formation of laminates containing an additive matter. Such additive matters can comprise various materials including gels, liquids, superabsorbent polymers, particulate additives and the like. The present invention can in one embodiment be used to form absorbent cores for use in disposable products such as diapers and sanitary napkins.

Sanitary napkins used in feminine hygiene are absorbent items worn by women to recover undesirable bodily discharges. These absorbent articles are typically comprised of an absorbent core sandwiched between layers of woven or non-woven materials.

Generally, diapers comprise an absorbent insert or patch and a chassis, which, when the diaper is worn, supports the insert proximate a wearer's body. Additionally, diapers may include other various patches, such as tape tab patches, reusable fasteners and the like. The raw materials used in forming a representative insert are typically cellulose pulp, tissue paper, poly, nonwoven web, an acquisition layer, and elastic, although application specific materials are sometimes utilized. Usually, most of the insert raw materials are provided in roll form, and unwound and applied in assembly line fashion.

In the creation of a diaper (and, oftentimes also in conjunction with feminine hygiene products), multiple roll-fed web processes are typically utilized.

To create a common absorbent insert, the cellulose pulp is unwound from the provided raw material roll and pulverized by a pulp mill. Discrete pulp cores are formed by a core forming assembly and placed on a continuous tissue web. Optionally, super-absorbent powder may be added to the pulp core. The tissue web is wrapped around the pulp core. The wrapped core is debulked by proceeding through a calendar unit, which at least partially compresses the core, thereby increasing its density and structural integrity. After debulking, the tissue-wrapped core is passed through a segregation or knife unit, where individual wrapped cores are cut. The cut cores are conveyed, at the proper pitch, or spacing, to a boundary compression unit.

While the insert cores are being formed, other insert components are being prepared to be presented to the boundary compression unit. For instance, the poly sheet is prepared to receive a cut core. Like the cellulose pulp, poly sheet material is usually provided in roll form. The poly sheet is fed through a splicer and accumulator, coated with an adhesive in a predetermined pattern, and then presented to the boundary compression unit. In addition to the poly sheet, which may form the bottom of the insert, a two-ply top sheet may also be formed in parallel to the core formation. Representative plies are an acquisition web material and a nonwoven web material, both of which are fed from material rolls, through a splicer and accumulator. The plies are coated with adhesive, adhered together, cut to size, and presented to the boundary compression unit. Therefore, at the boundary compression unit, three components are provided for assembly: the poly bottom sheet, the core, and the two-ply top sheet.

A representative boundary compression unit includes a die roller and a platen roller. When all three insert components are provided to the boundary compression unit, the nip of the rollers properly compresses the boundary of the insert. Thus, provided at the output of the boundary compression unit is a string of interconnected diaper inserts. The diaper inserts are then separated by an insert knife assembly and properly oriented. At this point, the completed insert is ready for placement on a diaper chassis.

A representative diaper chassis comprises nonwoven web material and support structure. The diaper support structure is generally elastic and may include leg elastic, waistband elastic and belly band elastic. The support structure is usually sandwiched between layers of the nonwoven web material, which is fed from material rolls, through splicers and accumulators. The chassis may also be provided with several patches, besides the absorbent insert. Representative patches include adhesive tape tabs and resealable closures.

The process utilizes two main carrier webs; a nonwoven web which forms an inner liner web, and an outer web that forms an outwardly facing layer in the finished diaper. In a representative chassis process, the nonwoven web is slit at a slitter station by rotary knives along three lines, thereby forming four webs. One of the lines is on approximately the centerline of the web and the other two lines are parallel to and spaced a short distance from the centerline. The effect of such slicing is twofold; first, to separate the nonwoven web into two inner diaper liners. One liner will become the inside of the front of the diaper, and the second liner will become the inside of the back of that garment. Second, two separate, relatively narrow strips are formed that may be subsequently used to cover and entrap portions of the leg-hole elastics. The strips can be separated physically by an angularly disposed spreader roll and aligned laterally with their downstream target positions on the inner edges of the formed liners.

After the nonwoven web is sliced, an adhesive is applied to the liners in a predetermined pattern in preparation to receive leg-hole elastic. The leg-hole elastic is applied to the liners and then covered with the narrow strips previously separated from the nonwoven web. Adhesive is applied to the outer web, which is then combined with the assembled inner webs having elastic thereon, thereby forming the diaper chassis. Next, after the elastic members have been sandwiched between the inner and outer webs, an adhesive is applied to the chassis. The chassis is now ready to receive an insert.

To assemble the final diaper product, the insert must be combined with the chassis. The placement of the insert onto the chassis occurs on a placement drum or at a patch applicator. The inserts are provided to the chassis on the placement drum at a desired pitch or spacing. The generally flat chassis/insert combination is then folded so that the inner webs face each other, and the combination is trimmed. A sealer bonds the webs at appropriate locations prior to individual diapers being cut from the folded and sealed webs.

Generally, disposable undergarments such as pants-type diapers are made up of two nonwoven layers of material with elastic strands of material placed between the two nonwoven layers of material thus creating an elastic web laminate. The layers of material are continuous sheets of material that are eventually cut into individual undergarment lengths. The elastic strands may be arranged and cut so that specific areas of the undergarment are free of elastic tension or forces. An absorbent pad, often contained within an insert or core is then also placed into the pants-type diaper product.

To insure the pants-type diaper retains a proper shape and to hold all of the added layers of the diaper, reinforcing layers and backing materials are normally added to the continuous sheets of material, with the reinforcing layers corresponding to the cut elastic strands of each individual blank. Each of these layers needs to be adhesively joined at some point in the manufacturing process to the elastic web laminate to form the completed undergarment.

Typically, absorbent fibrous material used to create an absorbent core is composed of cellulose wadding or cellulosic wood pulp material commonly referred to as "fluff", although a mixture of natural and synthetic fibers is also used. An absorbent core is typically composed of wood pulp fluff is typically formed by employing conventional air laying techniques.

These absorbent cores have had their total absorbency improved greatly by the addition of super absorbent material, or super absorbent polymer (SAP) to the commonly used absorbent fibrous materials.

The ability of these absorbent cores to manage the typical surges of liquid flow is heavily dependent on the proper distribution of super absorbent material within the absorbent fluff. When most super absorbent materials absorb aqueous fluids, they swell substantially, often to double their dry dimensions or more at saturation. As these super absorbent materials absorb fluid and swell, they generally become a gelatinous mass.

There has been a trend in reducing the bulk of diapers, in attempts to make them more like underwear and to take up less shelf space in retailer's outlets. Generally, the thinner the diaper, the higher the concentration of super absorbent material required to produce the same level of absorbency. High levels of super absorbent material, however, tend to be more difficult to control and to maintain in position. The present invention assists in this task.

In conventional core forming processes, three-dimensional fluff receiving pockets rotate about a vacuum drum. The pockets typically include baffles and screens which permit airflow through the pockets. The fluff is applied to the fluff receiving pockets entrained in air applied to the pockets. The vacuum attracts the fluff to a screen-like mesh that forms the pockets. The fluff is retained by the pockets, and the amount of fluff builds up from the screen forming the pocket. However, some fluff passes through the screen of the pockets and into the vacuum stream that is drawing the fluff into the pocket. Thus, some fluff undesirably becomes entrained in the vacuum stream.

In the core forming process, it is required to balance the amount of air urging the fluff towards the core forming pocket and the amount of vacuum used to retain the fluff within the pocket. Machine processes have become more complex as speeds of machines have increased, so air handling systems used in this process have greater demands placed on them. For instance, if the machine is running faster, pulp is required to be delivered to the core forming pocket quicker, necessitating a greater air flow to the pocket. To deliver more pulp to the pocket, more vacuum is required to retain the pulp within the pocket. One complication is in achieving optimum balance between air in to the pocket and vacuum applied to the back side of the pocket.

Imbalance between the amount of air supplying pulp to the core forming pocket and vacuum applied to the back of the pocket, holding the fluff in, causes puffs of fluff to escape forming chamber. Conventional core forming technology allows for limited adjustability to try and achieve the optimum balance between air in and vacuum. The largest air delivery is from fiberizing mill which supplies fluff and blows the fluff into the core forming chamber.

Another source of air into forming process is from the dust collection equipment, which returns collected fluff from the vacuum stream to the core forming drum. Beginning with fluff that passes through the core forming pocket, the vacuum stream leads the fluff within the vacuum stream to the dust collection unit. A filter within the dust collection unit captures this fluff, this fluff is removed from the filter, and recirculated into the core forming process. Typically, this vacuum stream is fed into a drum filter housing, such as described in U.S. Patent No. 5,679,136, commercial embodiments of which are available from the Osprey Corporation, and which is incorporated herein by reference.

The process of removing dust off of the filter uses a high volume of air. It must collect all of the dust, and return the fluff dust to core forming ducting. When the diaper making machine is stopped, it is undesirable to return fluff to the core forming process, because the core forming process is on hold until regular operation resumes. Ordinarily, in a shut down situation, the vacuum off of the filter in the dust collection unit is still operating, and collected fluff or dust gets diverted to a main drum filter. This process forms a closed loop recirculation while machine is idle. However, components of the system, such as a nozzle fan, end up beating the recirculated pulp into a fine powder, and this is undesirable because the powdered fluff lacks fluid retaining characteristics.

Other sources of air delivery to the forming chamber include the SAP delivery system, and air-bleed openings in the forming chamber itself.

### Summary of the Invention

This invention relates to formation of laminates containing an additive matters such as gels, liquids, superabsorbent polymers, particulate additives and the like. The present invention can be used to form absorbent cores for use in disposable products such as diapers and sanitary napkins.

In general terms, the invention comprises several variations of discrete product formation techniques. It is desired to create a new type of laminate which can be used, for instance, as an absorbent core in diaper manufacturing or other disposable product manufacturing. Alternatively, with several different additive types, such as gels, superabsorbent polymers, particulate additives and the like, a wide number of new discrete products can be created for a wide variation of uses.

In one embodiment, the present invention can be used to form absorbent cores for use in disposable products such as diapers and sanitary napkins, the absorbent containing no pulp, or very little pulp.

A laminate comprising a first material layer, a second material layer having a plurality of void spaces carried by said first material layer, an additive material carried in said void spaces, and a third material layer coupled to said first and second layers to form a laminated material is disclosed. Said second material layer can comprise a scrim material, selected from the group of bonded, unbounded, woven, polymer, non-polymer, and textile, and can be formed as woven or non-woven netting. The scrim material can contain a wicking component. The void spaces of the scrim material can comprise square and round voids, or voids of any desired shape. Additive materials can be introduced into the void spaces of the scrim, such as super absorbent polymer, cellulosic fluff, a mixture of super absorbent polymer and cellulosic fluff, or fluids or gels. The additive material can be distributed and carried in selected void spaces to define a pattern of additive material. The first and said second material layers can comprise nonwoven material.

A five or more layer laminate is also disclosed, said first material layer, said second material layer having a plurality of void spaces, a fourth layer comprising intermediate web layer overlying said second material layer, a fifth layer comprising an additional material layer having a plurality of void spaces overlying said intermediate web layer, additive material carried in said void spaces of said fifth layer, and said third material layer overlying said fifth layer.

A method of forming a laminate comprising supplying a first material layer, supplying a second material layer carried by said first material layer, said second material layer comprising a plurality of void spaces, coupling said first material to said second material layer, supplying an additive material to said void spaces of said second material layer, and supplying a third material layer and coupling said third material layer to said first and second material layers to create a laminated material is disclosed.

SAP or other additives can be introduced by at least one of vibration, gravity, ultrasonic agitation, and vacuum to said first material layer through said void spaces. Additives can also comprise a gel or liquid, which can be supplied by a dispensing nozzle. Additives can be supplied intermittently or continuously. The scrim layer or layers can also be stretched in at least one of a cross machine direction or a machine direction prior to coupling said first material to said second material layer.

### Brief Description of the Drawings

Fig. 1a is a perspective view of one embodiment of a laminate produced in accordance with the present invention, the laminate containing a continuous scrim material carried between at least two outer material layers, the scrim material containing between its voids an additive material;
Fig. 1b is a perspective view of a second embodiment of a laminate produced in accordance with the present invention, the laminate containing a discontinuous scrim material carried between at least two outer material layers, the scrim material containing between its voids an intermittently applied additive material;
Fig. 1c is a perspective view of a third embodiment of a laminate produced in accordance with the present invention, the laminate containing a continuous scrim material carried between at least two outer material layers, the scrim material containing between its voids an intermittently applied additive material;
Fig. 2 is a schematic view of an apparatus for forming a laminate produced in accordance with the present invention, the laminate containing a continuous scrim material carried between at least two outer material layers, the scrim material containing between its voids an additive material;
Fig. 3 is a cross-sectional view of a laminate produced in accordance with the present invention, the laminate containing a continuous scrim material carried between at least two outer material layers, the scrim material containing between its voids an additive material;
Fig. 4 is a schematic view of an additional apparatus for forming a laminate produced in accordance with the present invention, the laminate containing a continuous scrim material carried between at least two outer material layers, the scrim material containing between its voids an additive material.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention.

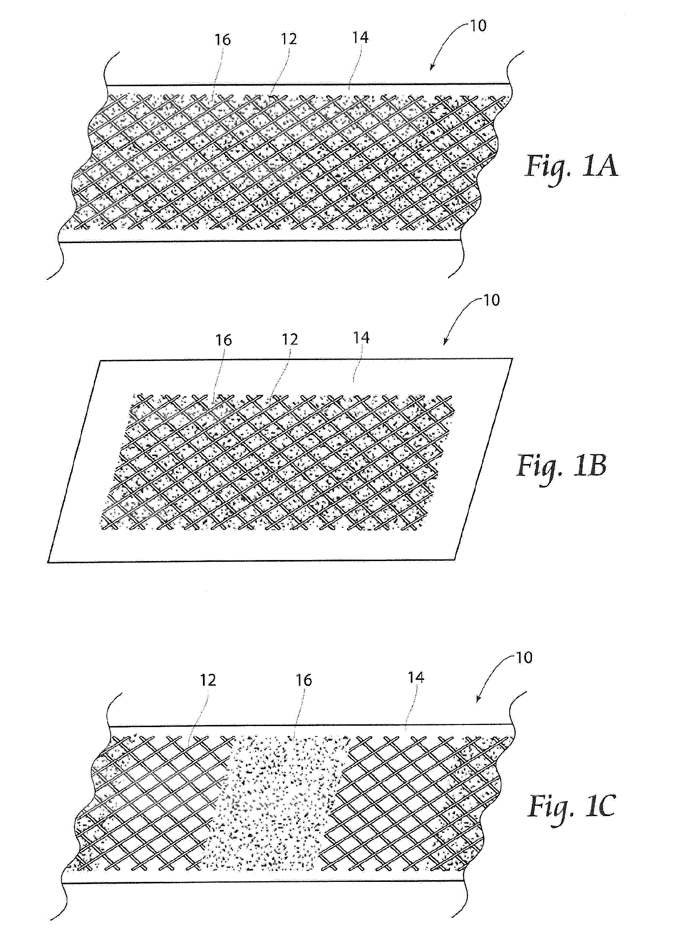

Referring now to Fig. 1a, a perspective view of one embodiment of a laminate 10 produced in accordance with the present invention is shown. The laminate 10 contains a continuous scrim material 12 carried between at least two outer material layers 14 and 24, the scrim material 12 containing between its voids an additive material 16. A border either around or on two sides of the laminate can be provided in which there is no additive 16 or scrim material 16.

Scrim material 12 can be bi-component, i.e. not have to be a uniform scrim either of material or shape. Void spaces in scrim material 12 could be combination of round and square for example, or polymer components coupled with fibrous components such as textile and yarn. Scrim material could be formed as a woven netting, for example, and could provide a wicking transfer means from one void to another or contain a wicking component.

A wide variation of additives 16 can be used. For instance, powders, gels, Superabsorbent Polymers commonly used in diaper manufacturing (SAP), particulates, and flakes can be used based on end-user preference or product specifications. In that instance, void spaces in the scrim 12 are provided with liquid to fill them.

An additional embodiment (not shown) could be a five (or more) layer laminate comprising a carrier web, scrim layer, intermediate web, scrim layer and another carrier web (or variations or additions thereto) is possible.

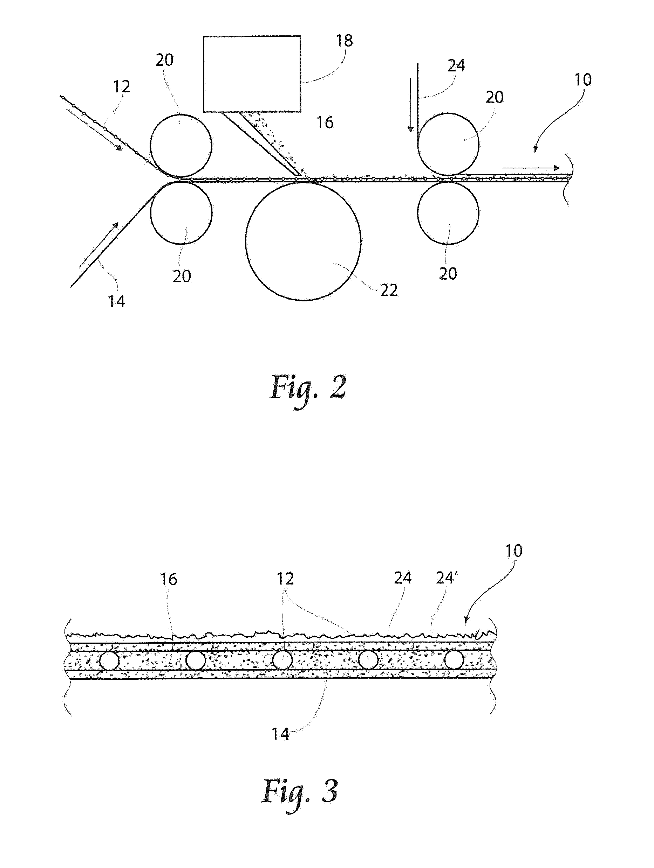

Regarding the scrim 12 material itself, this material can also vary widely. For instance, different shaped void spaces (diamond, rectangular, square, circular etc) can be used, and different sized void spaces can also be used (e.g., ¼" - 1" diameter). The thickness of the scrim can vary based on end-user or application preference, and the cross-sectional shape of the scrim material can also vary (e.g., oblong, circular or rectangular). The scrim 12 can either be elastic or inelastic, and incoming scrim (see Fig. 2) could be either applied to the system in an elongated state or applied in a non-elongated state. The elongation could be a combination of machine direction and cross machine direction elongation, or a combination of elongated and not elongated material. The scrim 12 can consist in itself of or contain heat activated adhesive whereby running it between heated rolls adheres the opposing layers. The scrim 12 can contain adhesive stands in one direction and elastic or non-elastic strands in the other direction.

Referring now to Fig. 1b, a perspective view of second embodiment of a laminate 10 produced in accordance with the present invention is shown. In this embodiment, the laminate contains a discontinuous scrim material 12 carried between at least two outer material layers 14 and 24 (see Fig. 2), the scrim material 12 containing between its voids an intermittently applied additive material 16 to create a patch area of material applied with the scrim 12/additive 16 combination.

Intermittent application of stretchable material is taught in commonly owned U.S. Patent Application No. 12/925,570 and is incorporated herein by reference. Additionally, slip/cut systems can be used to provide intermittent webs into a web processing system.

Referring now to Fig. 1c, a perspective view of a third embodiment of a laminate produced in accordance with the present invention is shown. In this embodiment, the laminate 10 contains a continuous scrim material 12 carried between at least two outer material layers 14 and 24 (see Fig. 2), the scrim material 12 containing between its voids an intermittently applied additive material 16.

Referring now to Fig. 2, a schematic view of an apparatus for forming a laminate 10 produced in accordance with the present invention is shown. The laminate is formed by first supplying a continuous web of material 14 (or two different webs as desired). Scrim 12 is introduced to the web 14, and passed to two closed spaced, preferably heated compression rollers 20. The first carrier web 14, and the scrim 12 are bonded between compression rollers 20, which are preferably heated. Alternatively, an adhesive could be introduced to the scrim itself which would create a tacky or heat activated scrim coupled to the web 14. Bonding can also be accomplished by a combination of any of adhesives, heat, heat-activated adhesive, ultrasonic, RF energy activation, microwave activation, UV activation, or pressure.

Next, the coupled scrim 12 and carrier web 14 are passed to roll 22 which is preferably a vacuum roll. The roll 22 preferably wraps the partial laminate for a portion of its roll 22 periphery. Roll 22 preferably serves as a control measure for the web, and allows for a precise placement for additive material. Roll 22 could be a vacuum conveyor or some other vacuum transport system, or a different type of conveying system.

From additive source 18, the coupled scrim 12 and carrier web 14 are provided and overlain in a pattern as desired with additive 16. In the instance of a solid particulate layer for additive 16, a vibratory tray or the like can be used to apply additive 16 to a moving web underneath. In the case of a gel or liquid, a continuous or intermittent dispensing nozzle or series of nozzles could be used to introduce the additive 16. If settling of additive 16 is desired, gravity means, vibratory means, electrical charging (polarity attraction of SAP), or ultrasonic agitation, could be used in addition to or in place of vacuum.

Next, the webs 12/14 are overlain with a second incoming web or webs 24 prior to passing through a second set of preferably heated compression rollers 20. Web types can vary, and typically for disposable products can include non-woven webs, woven webs, a curly fiber type material, or an acquisition layer for evenly distributing liquids throughout. An acquisition layer could also be a timed patch applied to the carrier web upstream of the introduction to the second pair of compression rollers, as taught with regard to intermittent web application above.

The formed laminate 10 is then passed downstream for further processing as desired, such as severing the laminate 10, or severing and coupling the laminate 10 to an additional laminate.

Still referring to Fig. 2, in an alternative embodiment top and bottom webs 12 and 14 could be a single web that wraps from the bottom (not shown) to cover scrim 12 after introduction of additive matter.

Referring now to Fig. 3, a cross-sectional view of a laminate 10 produced in accordance with the present invention is shown. The laminate contains scrim material 12 carried between at least two outer material layers 14 and 24 and 24', the scrim material containing and carrying between its voids additive material 16.

Several variations of the product and procedure for making it are available. For instance, additive matter could also be a fluid (gas or liquid) in addition to absorbent core material, including superabsorbent polymers. The additive matter could be a combination of additives, such as particles, flakes, gel, and any combination thereof. Another alternative is that scrim construction could be bonded or un-bonded, woven, polymer based, non-polymer based, textile(fabric) based, non-textile based, or any combination of the foregoing. Continuous or intermittent application of scrim, additive, using one or more application methods (multiple additive matter applicators, multiple scrim introducers, etc. could all be used.

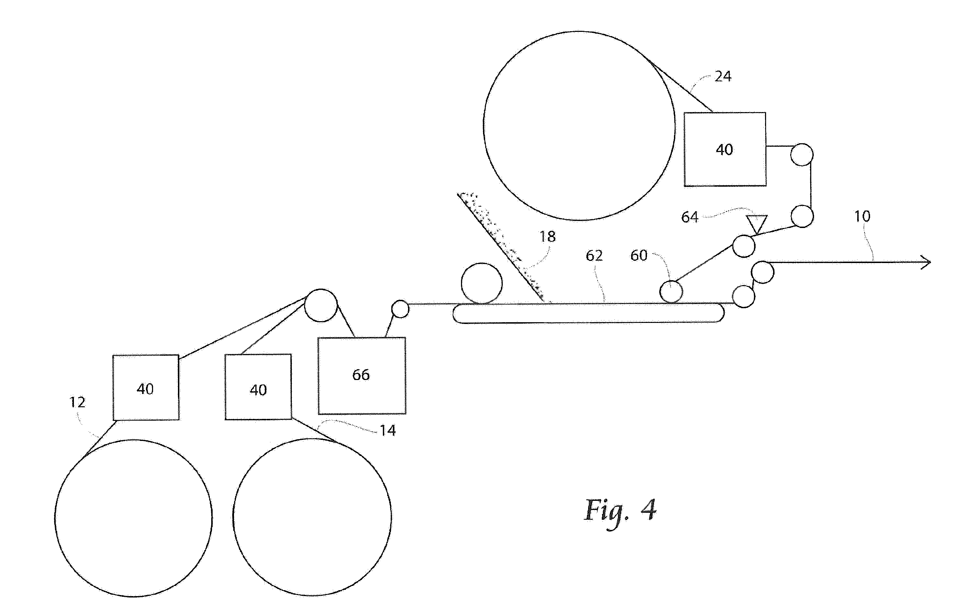

Referring now to Fig. 4, a schematic view of an additional apparatus for forming a laminate 10 produced in accordance with the present invention is shown. The laminate 10 is formed by introducing a scrim material to tension control unit 40, and introducing a material layer 14 into tension control unit 40. Both of these webs are introduced into bonding unit 66, which in the case of a heat activated adhesive scrim 12, could be a heating unit 66. Next, this combined web 12/14 is introduced to conveyor 62, which is preferably a vacuum conveyor. SAP is introduced through introduction unit 18, which as previously mentioned can be vibratory, gravity fed, etc. SAP is carried by material 14 and the SAP becomes carried between strands of the scrim 12. Next, another material layer 24 is fed through tension control unit 40 and adhesive unit 64 applies adhesive to the material 24. This layer is then applied to the top of the combined web 12/14 carrying SAP 18 and the combined web is fed to combining unit 60, and fed downstream for further processing as desired. Combining unit 60 can also be a combination of any of adhesives, heat, heat-activated adhesive, ultrasonic, RF energy activation, microwave activation, UV activation, or pressure.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A laminate comprising:
a first material layer;
a second material layer having a plurality of void spaces carried by said first material layer;
an additive material carried in said void spaces;
and a third material layer coupled to said first and second layers to form a laminated material.

2. A laminate according to claim 1, said second material layer comprising a scrim material.

3. A laminate according to claim 2, said scrim material selected from the group of bonded, unbounded, woven, polymer, non-polymer, and textile.

4. A laminate according to claim 2, said scrim material formed as woven netting.

5. A laminate according to claim 2, said scrim material containing a wicking component.

6. A laminate according to claim 1, said void spaces comprising at least one of square and round voids.

7. A laminate according to claim 1, said additive comprising super absorbent polymer.

8. A laminate according to claim 1, said additive comprising cellulosic fluff.

9. A laminate according to claim 1, said additive comprising a mixture of super absorbent polymer and cellulosic fluff.

10. A laminate according to claim 1, said additive comprising a fluid.

11. A laminate according to claim 1, said additive material carried in selected void spaces to define a pattern of additive material.

12. A laminate according to claim 1, said first and said second material layers comprising nonwoven material.

13. A laminate according to claim 1, said laminate further comprising said first material layer, said second material layer having a plurality of void spaces, a fourth layer comprising intermediate web layer overlying said second material layer, a fifth layer comprising an additional material layer having a plurality of void spaces overlying said intermediate web layer, additive material carried in said void spaces of said fifth layer, and said third material layer overlying said fifth layer.

14. A method of forming a laminate comprising:
supplying a first material layer;
supplying a second material layer carried by said first material layer, said second material layer comprising a plurality of void spaces;
coupling said first material to said second material layer;
supplying an additive material to said void spaces of said second material layer;
supplying a third material layer and coupling said third material layer to said first and second material layers to create a laminated material.

15. A method according to claim 14, said first and said second material layers comprising nonwoven material.

16. A method according to claim 14, said additive material supplied by at least one of vibration, gravity, ultrasonic agitation, and vacuum to said first material layer through said void spaces.

17. A method according to claim 14, said additive material comprising at least one of a gel or liquid.

18. A method according to claim 17, said additive material supplied by a dispensing nozzle.

19. A method according to claim 14, said additive material supplied intermittently.

20. A method according to claim 14, said method further comprising stretching said second material in at least one of a cross machine direction or a machine direction prior to coupling said first material to said second material layer.

21. A method according to claim 14, said coupling steps accomplished by at least one of adhesives, heat, heat-activated adhesive, ultrasonic, RF energy activation, microwave activation, UV activation, and pressure.
